# EUROPEAN PATENT APPLICATION

(11) **EP 1 211 309 A1**
(43) Date of publication of application: **05.06.2002**
(21) Application number: 00953532.9
(22) Date of filing: 21.08.2000
(51) Int. Cl.: C12M 3/00, C12N 7/00

(54) **METHOD AND APPARATUS FOR PROLIFERATING HEPATITIS VIRUS**

(30) Priority: 20.08.1999 JP 23364799
(71) Applicant: Miyamura, Tatsuo, Shinju-ku, Tokyo 162-8640 (JP); Nagamori, Seishi, Minato-ku, Tokyo 105-0014 (JP)
(72) Inventor: NAGAMORI, Seishi, Minato-ku, Tokyo 105-0014 (JP)
(74) Representative: Cresswell, Thomas Anthony
(86) International application number: JP0005582
(87) International publication number: WO0114517

(57) **Abstract**

A method for proliferating a hepatitis virus such as HCV and an apparatus therefor. A method of proliferating cells (for example, hepatocytes), which are less adhesive to a carrier, in a large amount over a long time. More particularly, the above method comprises infecting hepatocytes, which are maintained in a radial flow type hepatocyte bioreactor consisting of a main bioreactor unit containing the hepatocytes carried on a particulate porous carrier and a liquid culture medium flown from the periphery of the main bioreactor unit toward the center thereof, with a hepatitis virus; continuously flowing the liquid culture medium from the periphery of the main bioreactor unit toward the center thereof; and thus proliferating the hepatitis virus in the hepatocytes thus infected.

SEQUENCE LISTING

<110> Seishi NAGAMORI

<120> Method for Proliferating Hepatitis Virus and Apparatus Therefor

<130> JA904421

<150> JP 11-233647
<151> 1999-08-20

<160> 5

<210> 1
<211> 20
<212> DNA
<213> Artificial Sequence
<223> primer used for synthesizing cDNA of hepatitic C virus by reverse
transcription

<400> 1
aacactactc ggctagcagt          20

<210> 2
<211> 20
<212> DNA

<213> Artificial Sequence
<223> primer used for amplifying by PCR hepatitis C virus cDNA

<400> 2
ctgtgaggaa ctactgtctt          20

<210> 3
<211> 20
<212> DNA
<213> Artificial Sequence
<223> primer used for amplifying by PCR hepatitis C virus cDNA

<400> 3
aacactactc ggctagcagt          20

<210> 4
<211> 20
<212> DNA
<213> Artificial Sequence
<223> primer used for amplifying by PCR hepatitis C virus cDNA

<400> 4
ttcacgcaga aagcgtctag          20

<210> 5
<211> 20

<212> DNA
<213> Artificial Sequence
<223> primer used for amplifying by PCR hepatitis C virus cDNA

<400> 5
gttgatccaa gaaaggaccc          20

## Description

### Technical Field

The present invention relates to a method for proliferating hepatitis viruses for example hepatitis type C virus (HCV), and an apparatus therefor.

### Background of the Invention

The cDNA of HCV was cloned in 1989 and since then, the structure and processing mechanism thereof have been elucidated, using various expression systems actively. Consequently, very effective diagnostic systems have been developed, so that post-blood transfusion hepatitis due to HCV has virtually been under control currently in Japan.

As described above, the overall profile of HCV has increasingly been revealed, but research works on HCV at the genetic level have preceded. Therefore, fundamental research works for the elucidation of the biology and oncogenesis mechanism of HCV, including for example virus replication, particle formation and mutagenesis, have not made a progress yet or therapeutic methods with medications such as HCV vaccine, protease inhibitors or antisense have not yet been developed. This is due to the absence of any HCV proliferation system outside biological organisms. It is very difficult to proliferate HCV in hepatocyte during culture. No success in such proliferation has been reported yet. Therefore, currently, not any approach except for the use of chimpanzee now exists for the research works. However, the use costs very expensive, involving problems in terms of individual difference and reproducibility. Additionally, the use has some limitation from the respect of animal protection. Based on such background, it has been desired to establish a proliferation system of hepatitis viruses such as HCV, using a cell under culture, independently on clinical trials or animal experiments.

### Disclosure of the Invention

It is a purpose of the invention to provide a method for proliferating hepatitis viruses such as HCV, using hepatocyte under culture. Further, the invention provides a method for culturing a cell with low adhesivity, such as hepatocyte, outside biological organisms, at a mass scale in a three dimension for a long term. Still further, the invention provides a method for proliferating hepatitis viruses such as HCV.

The inventors have found as the outcome of intensive research works that hepatitis viruses such as HCV can be proliferated by culturing hepatocyte in a culture apparatus for allowing a liquid culture medium to flow in a culture vessel placing therein a carrier capable of immobilizing thereon the hepatocyte, for example a radial flow type bioreactor, allowing the hepatocyte to be infected with HCV, and continuing the culture of the hepatocyte.

In other words, the invention relates to a method for proliferating a cell with low adhesivity, such as hepatocyte, at a mass scale at a high efficiency for a long term, where the method is characterized by making a liquid culture medium flow around the periphery of a carrier being placed in a culture vessel and capable of immobilizing thereon the cell with low adhesivity, for example hepatocyte, and then immobilizing the cell with low adhesivity on the carrier and thereby proliferating the cell. Further, the invention relates to a method for proliferating hepatitis virus, where the method is characterized by making a liquid culture medium flow around the periphery of a carrier being placed in a culture vessel and capable of immobilizing thereon a cell with low adhesivity, for example hepatocyte, and then immobilizing the cell with low adhesivity on the carrier and thereby proliferating the cell to permit the cell under culture to be infected with hepatitis virus.

In more detail, the invention relates to a method for proliferating hepatitis virus, where the method is characterized by allowing hepatocyte under culture in a culture apparatus which permits a liquid culture medium to flow from the periphery of a culture vessel placing therein a carrier immobilizing thereon the hepatocyte toward the center thereof, to be infected with hepatitis virus. Additionally, the invention relates to an apparatus for proliferating hepatitis virus, and a hepatitis virus proliferated by the method of the invention.

Still more specifically, the invention provides a method for proliferating hepatitis virus, where the method is characterized by allowing hepatocyte maintained in a radial flow type hepatocyte bioreactor to immobilize the hepatocyte on a particulate porous carrier therein and permit a liquid culture medium to flow from the periphery of the main bioreactor unit toward the center thereof, to be infected with hepatitis virus, and continuously allowing the liquid culture medium to flow from the periphery of the main. bioreactor unit toward the center thereof to culture the hepatocyte to thereby proliferate the infectious hepatitis virus in the hepatocyte. Still additionally, the invention provides an apparatus for proliferating hepatitis virus, the apparatus containing a radial flow type hepatocyte bioreactor including a main bioreactor unit capable of permitting a liquid culture medium to flow from the periphery thereof toward the center thereof, a liquid culture medium supply conduit for supplying a liquid culture medium to the periphery of the main bioreactor unit, a particulate porous carrier placed in the inside of the main bioreactor unit to immobilize hepatocyte thereon, and a liquid culture medium discharge conduit positioned in the inside of the main bioreactor unit, for discharging the liquid culture medium from the main bioreactor unit.

As the hepatitis virus of the invention, any hepatitis virus with which the cell of liver can be infected and which is thereby proliferated, is satisfactory and preferably includes so-called hepatitis viruses such as HCV, HBV, and HEV and Dengue fever virus with a potency of hepatocyte infection. As the cell with low adhesivity in accordance with the invention, additionally, cells with low adhesivity to carriers in a culture vessel, which have been believed to possibly involve difficulty in three-dimensional culture on the carriers according to the related art, are satisfactory and preferably include for example hepatocyte.

According to the method of the invention, hepatitis virus such as HCV can be recovered after the virus is proliferated in the discharged liquid culture medium. As described above, the invention provides for the first time a method for efficiently proliferating hepatitis virus such as HCV outside biological organisms, using the cell under culture.

Thus, the invention makes great contributions to the estimation of the effect of interferon for use in previous therapeutics and the development of vaccines against hepatitis virus such as HCV, the preparation of anti-HCV antibodies, and the development of therapeutic agents against hepatitis virus such as HCV, including for example protease inhibitors, polymerase inhibitors and antisense drugs, and the like.

### Brief Description of the Drawings

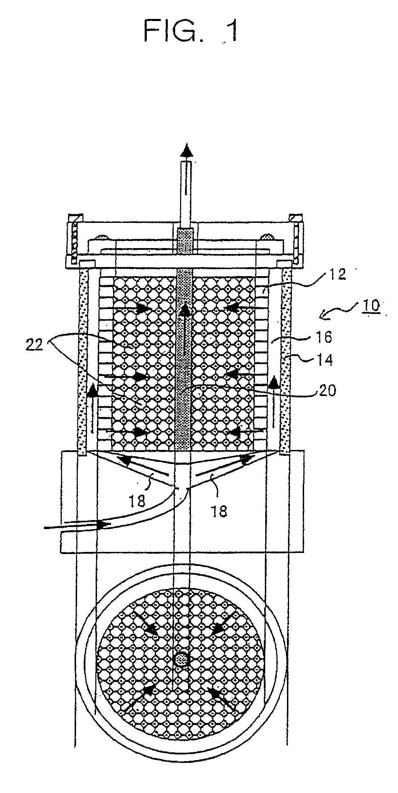

Fig. 1 depicts the longitudinal view and cross sectional view of a radial flow type bioreactor as one example of the culture apparatus for use in the method of the invention.

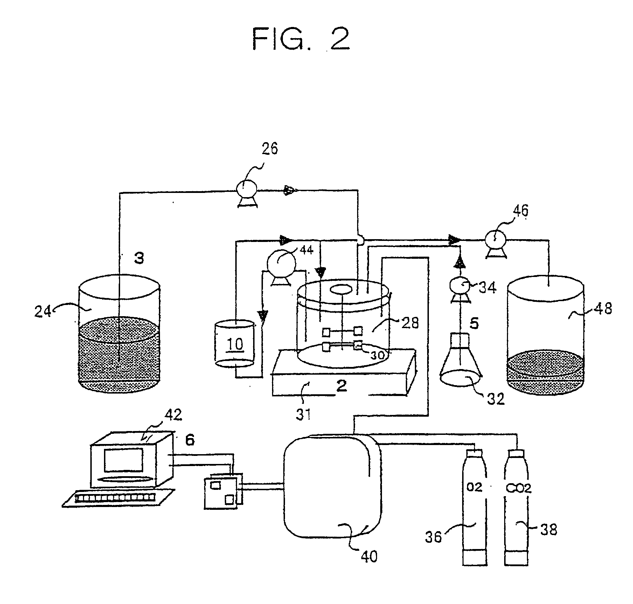

Fig. 2 is a view schematically depicting an example of the culture system of the invention.

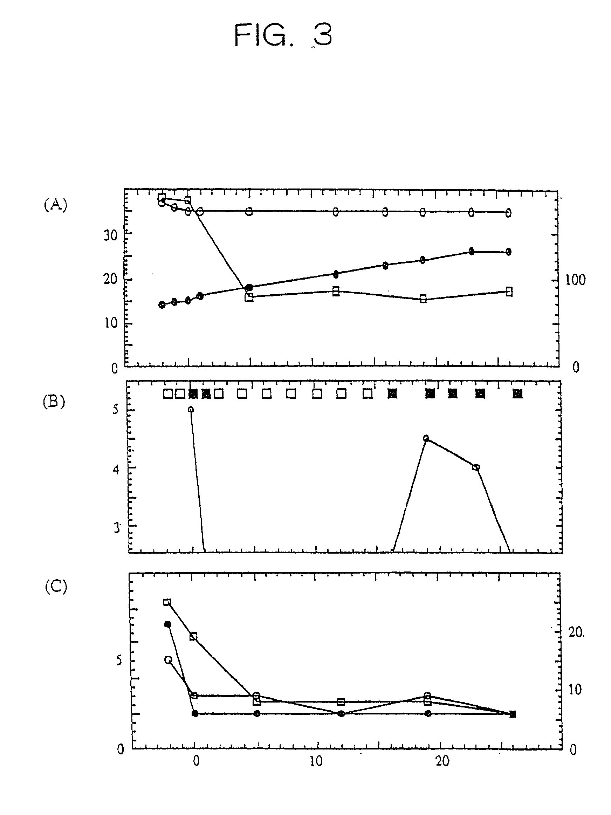

Fig. 3 depicts the changes of temperature (°C), oxygen concentration (ppm) and albumin concentration (µg/ml) (Fig. 3(A)) in a culture system using a serum-added liquid culture medium, the results of the detection of HCV in the discharged liquid culture medium (Fig. 3(B)), and the changes of GPT (IU/1), GOT (IU/1) and LDH (IU/1) in the liquid culture medium (Fig. 3(C)).
In Fig. 3 (A), open circle symbol (○) represents temperature (°C); solid circle symbol (●) represents oxygen concentration (ppm); and open square symbol (□) represents albumin concentration (µg/ml). The temperature (°C) and oxygen concentration (ppm) are depicted on the left scales, while the albumin concentration (µg/ml) is depicted on the right scale. The period in day is shown on the crosswise axis.
In Fig. 3 (B), open circle symbol (○) represents RNA titer (log₁₀ copy number/ml); open square symbol (□) represents that HCV core protein is negative; and solid square symbol (■) represents that HCV core protein is positive. The culture period in day is shown on the crosswise axis.
In Fig. 3 (C), solid circle symbol (●) represents GPT (IU/1); open circle symbol (○) represents GOT (IU/1); and open square symbol (□) represents LDH (IU/1). GPT (IU/1) and GOT (IU/1) are depicted on the left scales, while LDH (IU/1) is depicted on the right scale. The period in day is depicted on the crosswise axis.

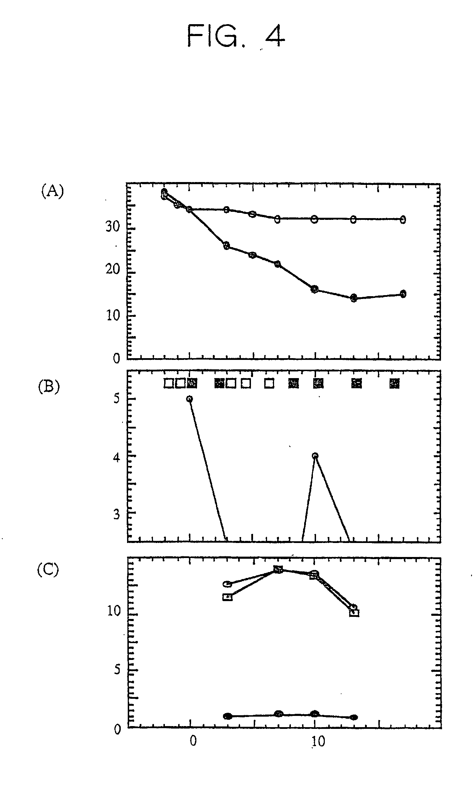

Fig. 4 depicts the changes of temperature (°C) and oxygen concentration (ppm) (Fig. 4(A)) in a culture system using a serum-free liquid culture medium, the results of the detection of HCV in the discharged liquid culture medium (Fig. 4(B)), and the changes of GPT (IU/1), GOT (IU/1) and LDH (IU/1) in the liquid culture medium (Fig. 4(C)).
In Fig. 4 (A), open circle symbol (○) represents temperature (°C) and solid circle symbol (●) represents oxygen concentration (ppm). The period in day is shown on the crosswise axis.
In Fig. 4 (B), open circle symbol (○) represents RNA titer (log₁₀ copy number/ml); open square symbol (□) represents that HCV core protein is negative; and solid square symbol (■) represents that HCV core protein is positive. The culture period in day is shown on the crosswise axis.
In Fig. 4 (C), solid circle symbol (●) represents GPT (IU/1); open circle symbol (○) represents GOT (IU/1); and open square symbol (□) represents LDH (IU/1). GPT (IU/1) and GOT (IU/1) are depicted on the left scales, while LDH (IU/1) is depicted on the right scale. The period in day is depicted on the crosswise axis.

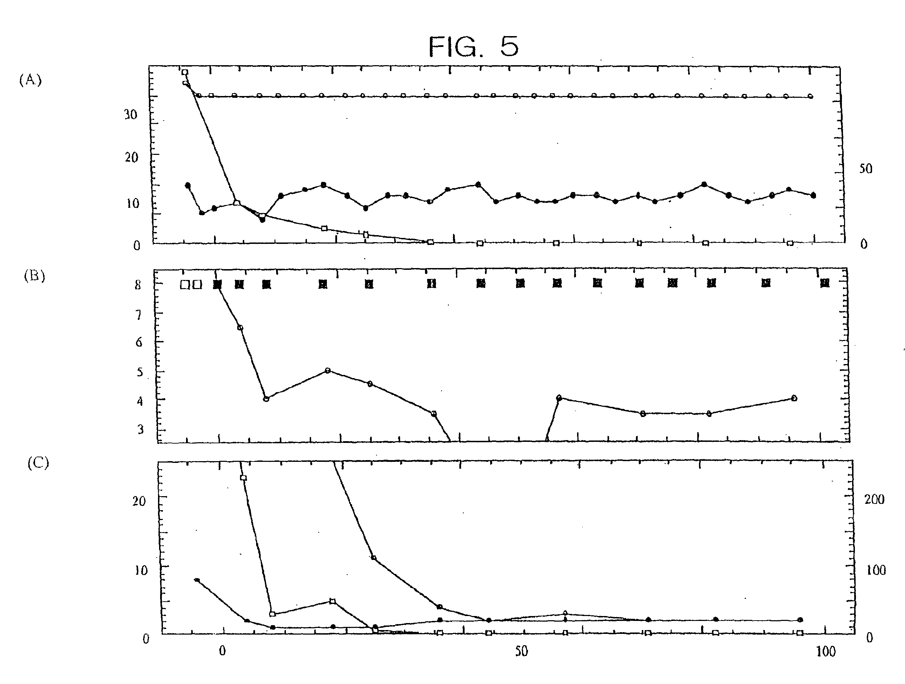

Fig. 5 depicts the changes of temperature (°C), oxygen concentration (ppm) and albumin concentration (µg/ml) (Fig. 5(A)) in a culture system using a serum-free liquid culture medium in case of transfection with infectious clone RNA of type 1a in the bioreactor during culture, the results of the detection of HCV in the discharged liquid culture medium (Fig. 5(B)), and the changes of GPT (IU/1), GOT (IU/1) and LDH (IU/1) in the liquid culture medium (Fig. 5(C)).
In Fig. 5 (A), open circle symbol (○) represents temperature (°C); solid circle symbol (●) represents oxygen concentration (ppm); and open square symbol (□) represents albumin concentration (µg/ml). The temperature (°C) and oxygen concentration (ppm) are depicted on the left scales, while the albumin concentration (µg/ml) is depicted on the right scale. The period in day is shown on the crosswise axis.
In Fig. 5 (B), open circle symbol (○) represents RNA titer (log₁₀ copy number/ml); open square symbol (□) represents that HCV core protein is negative; and solid square symbol (■) represents that HCV core protein is positive. The culture period in day is shown on the crosswise axis.
In Fig. 5 (C), solid circle symbol (●) represents GPT (IU/1); open circle symbol (○) represents GOT (IU/1); and open square symbol (□) represents LDH (IU/1). GPT (IU/1) and GOT (IU/1) are depicted on the left scales, while LDH (IU/1) is depicted on the right scale. The period in day is depicted on the crosswise axis.

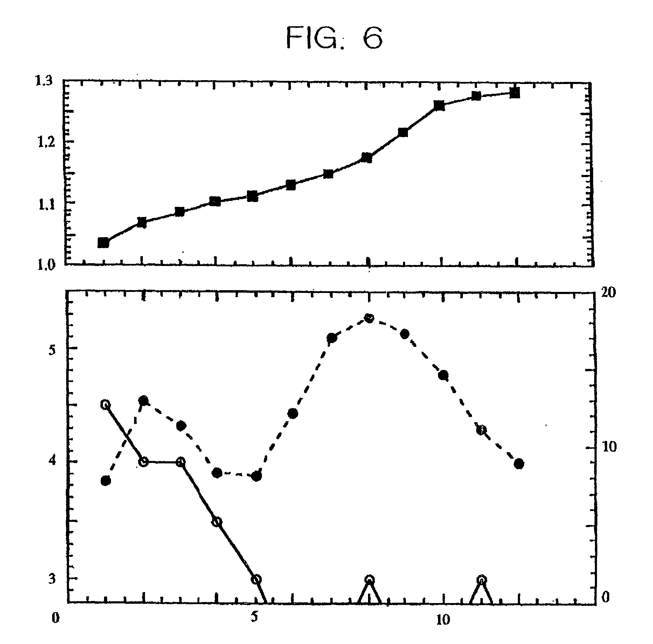

Fig. 6 depicts the results of the assay of HCV RNA and HCV core protein in each of the fractions fractionated from the liquid culture medium on day 96 post-transfection by a sucrose gradient method. In the graphs on the bottom in Fig. 6, open circle symbol (○) represents HCV RNA titer (log₁₀ copy number/ml) and solid open circle (●) represents the concentration (pg/ml) of HCV core protein. In the graph on the top in Fig. 6, solid square symbol (■) represents the density (g/ml) of the core protein.

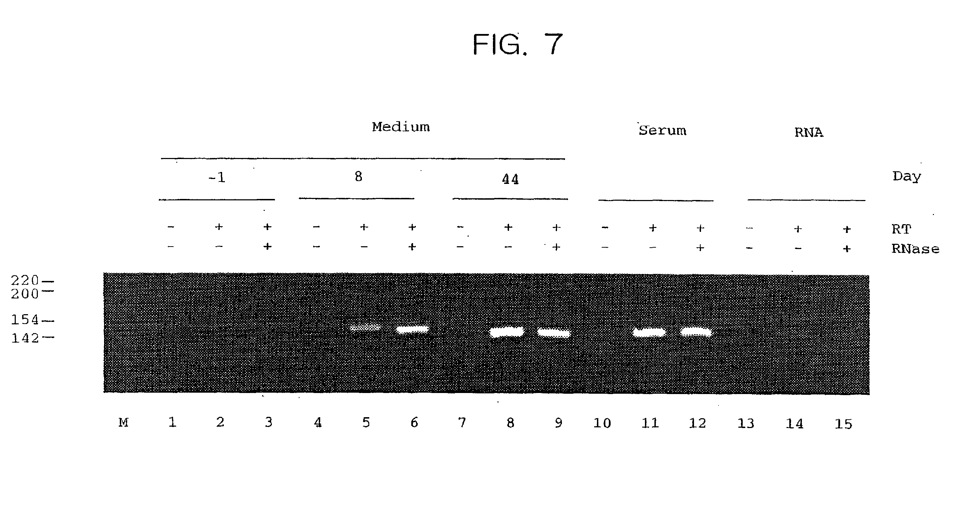

Fig. 7 depicts the results of the detection for a liquid culture medium with transfection with an infectious clone H77, on days 8 and 44 post-transfection, by RNase treatment and nest-RT PCR method. In Fig. 7, the liquid culture media on the previous day (day -1) of culture and on days 8 and 44 of culture, a control serum and a control RNA are shown from the left, individually in three lanes, namely a lane without RNase treatment (RNase-) and no nest-RT PCR treatment (RT-), a lane without RNase treatment (RNase-) but with nest-RT PCR treatment (RT+) and a lane with RNase treatment (RNase+) and nest-RT PCR treatment (RT+). The numerical figure on the top of Fig. 7 represents the period of culture in day; the symbols +- depicted on the top represent the presence (+) and absence (-) of the nest-RT PCR; and the symbols on the bottom represent the presence (+) and absence (-) of RNase treatment. The numerical figure in the longitudinal direction in Fig. 7 represents the number of bases (-mer).

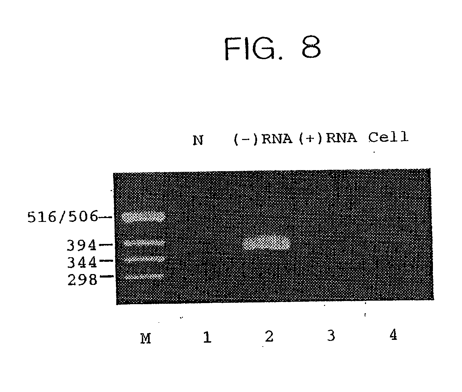

Fig. 8 is a figure depicting the results of the detection of minus-strand RNA in the cell on day 110 after the transfection with the infectious clone H77 by strand-specific RT-PCR method using a tag. In Fig. 8, M depicts marker; N in the lane 1 depicts control with cell (-) and in the absence of any negative strand RNA or positive strand RNA; (-) RNA in the lane 2 represents the case with addition of negative strand RNA, while (+) RNA in the lane 3 represents the case with addition of positive strand RNA. Cell in the lane 4 represents the case of HCV-infected cell cultured in RFB. The numerical figure in the longitudinal direction in Fig. 8 represents molecular weight.

### Best Mode for Carrying out the Invention

The culture apparatus for use in accordance with the invention is a culture apparatus capable of immobilizing hepatocyte on a carrier and allowing a liquid culture medium to flow in a culture vessel placing therein the carrier to thereby allow the liquid culture medium to consistently flow around the immobilized hepatocyte. The liquid culture medium may satisfactorily flow from any direction of the culture vessel. For example, the liquid culture medium may flow from the periphery of the culture vessel to the center thereof or from the bottom thereof to the top thereof or may flow in a combination mode thereof. Additionally, the carrier for immobilizing hepatocyte thereon may be any carrier capable of immobilizing culture cells thereon, with no specific limitation. Preferably, however, the carrier is a porous carrier. The material of the carrier includes glass and plastics.

Preferable one of the culture apparatus of the invention includes radial flow type bioreactor. One example of the preferable radial flow type bioreactor of the invention is described with reference to a drawing.

Fig. 1 schematically shows one example of the preferable radial flow type bioreactor of the invention. The upper figure in Fig. 1 shows the longitudinal view of the radial flow type bioreactor, while the lower figure therein shows the cross sectional view of the radial flow type bioreactor. Radial flow type bioreactor 10 includes cylindrical, main bioreactor unit (culture vessel) 12. The outer periphery wall of the main bioreactor unit 12 is made of a porous material with numerous through-holes, and through these through-holes, the liquid culture medium can flow from the outside of the main bioreactor unit into the inside thereof. The diameters of the through-holes are smaller than that of the carrier particle described below and are of a dimension enough for the liquid culture medium to be supplied sufficiently into the inside of the bioreactor 12. Generally, the diameters are about 20 to 80 µm, preferably. Cylindrical casing 14 is arranged for further enclosing the outer face of the main bioreactor unit 12 so that the cross section might form a concentric circle with the main bioreactor unit 12, while ring-like liquid culture medium supply conduit 16 is formed between the outer periphery wall of the main bioreactor unit 12 and the casing 14. On the bottom, the liquid culture medium supply conduit 16 is in communication with liquid culture medium supply pipe 18. In the center of the main bioreactor unit 12 is arranged liquid culture medium discharge pipe 20. The outer periphery wall of the liquid culture medium discharge pipe 20 is made of a porous material with numerous through-holes of the same dimension as for the main bioreactor unit 12, and the liquid culture medium can pass through the porous material but the carrier never passes through the porous material.

Inside the main bioreactor unit 12 are further placed numerous porous carriers 22 in particle. The material of the porous carrier preferably includes for example, but is not limited to, spherical porous glass bead. The diameter of the porous carrier includes, but is not limited to, preferably about 0.1 mm to 6 mm, particularly about 0.3 mm to 1.2 mm. Additionally, the pore diameter in the carrier preferably includes, but is not limited to, about 10 to 300 µm, particularly about 20 to 120 µm. Additionally, the void ratio in the carrier particle is preferably, but is not limited to, about 30 to 70%, particularly about 40 to 60%. Such porous glass bead is commercially available under the trade name of Siran from Schott Glasswerk Co. Ltd., Germany. The commercially available product can preferably be used. Additionally, the density of the carrier particle placed in the main bioreactor unit 12 is not specifically limited. Preferably, the carrier particle is poured into the main bioreactor unit 12, as much as possible, under gravity force.

The size of the radial flow type bioreactor is not specifically limited, The volume of the inside of the main bioreactor unit 12 is generally about 5 ml to several tens milliliters, but the volume may satisfactorily be outside the range.

Then, a method for culturing hepatocyte using the radial flow type bioreactor as described above will be described. The flow of the liquid culture medium is depicted with the arrow in Fig. 1. More specifically, the liquid culture medium is supplied through the liquid culture medium supply pipe 18 into the liquid culture medium supply conduit 16 from the bottom thereof on the outer periphery of the main bioreactor unit 12. The liquid culture medium flows upward in the liquid culture medium supply conduit 16 to enter from the numerous through-holes arranged on the outer wall of the main bioreactor unit 12 into the inside of the main bioreactor unit 12. Then, the liquid culture medium flows toward the center of the main bioreactor unit 12 therein, to enter from the numerous through-holes arranged on the liquid culture medium discharge pipe 20 into the liquid culture medium discharge pipe 20 and move upward in the liquid culture medium discharge pipe 20, so that the liquid culture medium is discharged from the top of the liquid culture medium discharge pipe 20 to the outside of the main bioreactor unit 12. Herein, the liquid culture medium is supplied from the bottom of the radial flow type bioreactor shown in Fig. 1. Because it is only required for the liquid culture medium to flow from the outer periphery of the main bioreactor unit 12 to the center thereof, the liquid culture medium is structurally supplied from the top of the liquid culture medium supply conduit 16. Additionally, a part of the discharged liquid culture medium is again recycled and supplied as a liquid culture medium, preferably. More specifically, a combination of fresh one of the liquid culture medium and the liquid culture medium recycled is preferably used as the liquid culture medium. The mix ratio thereof is preferably automatically controlled that the ratio of daily glucose consumption (g/day)/oxygen consumption (g/day) might be 0.5 to 15, particularly about 3 to 10.

The liquid culture medium herein used is of any composition, provided that the liquid culture medium can culture and proliferate hepatocyte. The liquid culture medium is satisfactorily a liquid culture medium containing essential components for cell culture, such as minerals, sugar, amino acid, peptide, vitamins, organic acid, nucleic acid, pH adjuster, and oxygen. For example, the minerals include NaCl, KCl, MgCl₂, MgSO₄, NaH₂PO₄, FeSO₄-7H₂O, ZnSO₄-7H₂O, CuSO₄-5H₂O and the like. The amino acid and peptide include L-aspartate hydrochloride salt, L-alanine, alanyl-L-glutamine, L-aspartic acid, L-glutamate, glycine, glycyl-L-glutamine, L-isoleucine, L-lysine, L-phenylalanine, L-serine, L-omithine, L-threonine, L-tryptophan, L-tyrosine, L-valine, insulin and the like. The sugar includes sugar and sugar alcohol, glycoside and the like, and includes for example D-glucose, D-mannose, D-galactose, and inositol. The organic acid includes free acids or organic acid derivatives such as esters, for example succinic acid, choline bitartrate, folic acid, sodium pyruvate and glycerophosphoric acid. The vitamins include pyridoxal hydrochloride, riboflavin and the like. The nucleic acid includes uridine and the like. The pH adjuster includes NaOH, carbonate gas, NaHCO₃ and the like.

Commercially available liquid culture media may be used as the liquid culture medium. Additionally, commercially available liquid culture media with addition of about 1 to 3 % of sera such as calf fetus serum, can also be used preferably. The oxygen concentration and pH in the liquid culture medium are preferably adjusted. The oxygen concentration in the liquid culture medium is preferably adjusted so that the oxygen concentration in the discharged liquid culture medium might be 1 ppm or more. More specifically, the oxygen supply per one milliliter volume of the main bioreactor unit (culture vessel) is preferably 0.025 to 0.75 ml/minute, particularly about 0.05 to 0.5 ml/minute. Additionally, the supply velocity of fresh one of the liquid culture medium is not specifically limited but is generally about 0.25 to 100 ml/day, preferably about 0.5 to 50 ml/day per one milliliter volume of the main bioreactor unit (culture vessel). Additionally, the circulation velocity of the liquid culture medium is not specifically limited but is generally 0.25 to 2.0 L/day, preferably about 0.5 to 1.0 L/day per one milliliter volume of the main bioreactor unit (culture vessel). Preferably, the liquid culture medium is adjusted to about pH 7.0, using aqueous sodium hydroxide solution, carbon dioxide and the like. Further, the temperature of the liquid culture medium is preferably adjusted to about 37°C or lower.

Hepatocyte proliferates while adhering to the surface of the porous carrier and to the inner surface of the pore in the porous carrier. The proliferating hepatocyte is immobilized on the surface of the porous carrier and the inner surface of the pore in the porous carrier and is additionally filled in the void in the porous carrier. The adhesion to and proliferation in the porous carrier of hepatocyte can be attained by adding hepatocyte to the liquid culture medium to prepare a hepatocyte suspension, and supplying the hepatocyte suspension as a liquid culture medium into the inside of the radial flow type bioreactor. When the hepatocyte suspension is supplied in the form of the liquid culture medium, the hepatocyte adheres to the surface of the porous carrier and to the inner surface of the pore in the porous carrier during the flow of the liquid culture medium in contact with the porous carrier, where the hepatocyte proliferates.

The density of hepatocyte added to the liquid culture medium at the start of culture is not specifically limited but is generally about 10⁵ to 10⁹ cells/ml, preferably about 10⁶ to 10⁷ cells/ml, while the total count of the hepatocyte added to the liquid culture medium is appropriately selected, depending on the volume of the main bioreactor unit. In case that the volume of the inside of the main bioreactor unit 12 is for example 200 ml, appropriately, the total count is generally about 10⁷ to 10¹⁰, preferably about 10⁵ to 10⁹. About three hours to 12 hours after the thorough distribution of the liquid culture medium with addition of hepatocyte throughout the main bioreactor unit, the flow of the liquid culture medium is stopped, while the efflux of hepatocyte from the main bioreactor unit is blocked, to promote the adhesion of hepatocyte onto the carrier.

Hepatocyte collected from human liver via autopsy and the like and proliferated by known plate culture methods and the like may be used as the hepatocyte, but so as to attain the proliferation and maintenance of hepatocyte in the bioreactor for a long term in a secure manner, preferably, an established cell line of hepatocyte is used. Established cell lines of hepatocyte are known. Any of the cell lines can be used. Preferable examples of the cell lines include, but are not limited, to FLC-4 (USP No. 5,804,441; deposited under accession No. FERM BP-5165 at the National Institute of Bioscience and Human-Technology), HepG2 (available from ATCC), Huh7 (available from Japanese Cancer Research Resources Bank (JCRB)), FLC-1, FLC-2, FLC-3, FLC-5, FLC-6 and FLC-7 (about these FLC series cell lines, see K. Fujise, S. Nagamori, H. Kameda et al., HEPATOLOGY, 8: 1425, 1988; Seishi Nagamori, et al., HUMAN CELL 1(1): 106, 115-118, 120, 123, 1988; Seishi Nagamori, et al., Current Therapy, 16: 158-162, 1998; Kawada, M. et al., In Vitro Cell. Dev. Biol., 34: 109-115, 1998; and Satoshi Hasumura, et al., Artificial Blood, 5, 33-37, 1997; and the like). The inventors have made comparative examinations about HCV proliferation potency of plural types of hepatocyte lines. It is preferable to use the FLC-4 cell because HCV proliferates best in FLC-4 cells. It is suggested that a certain host factor specifically stabilizing the mini-gene RNA of HCV to elevate the translation efficiency is present in FLC-4 cells.

When hepatocyte is cultured for 5 to 15 days after the supply thereof, generally, the hepatocyte sufficiently proliferates in a preferable state for infection with hepatitis virus. Hepatocyte proliferates in the main bioreactor unit to about 10⁸ cells/ml or more. In case that the volume of the main bioreactor unit 12 is 200 ml, hepatocyte proliferates up to about 2.9 ×10¹⁰ cells in total.

After hepatocyte proliferation, the hepatocyte is infected with hepatitis virus. By the method of the invention, any hepatitis viruses of type A, type B, type C, type D, type E, and type G can be proliferated. HCV is particularly preferable. According to the method of the invention, plural viruses of different types and plural viruses of different lines of an identical type can be proliferated simultaneously. Infection with hepatitis virus can be done by allowing a liquid culture medium for supply to contain the serum of a chronic hepatitis patient and then supplying it as the liquid culture medium. By directly adding the liquid culture medium containing hepatitis virus to hepatocyte in the main bioreactor unit, the infection possibility can be enhanced more. The volume of the serum of a hepatitis patient for supply is not specifically limited but is appropriately about 1/50- to 1/10-fold the volume of the main bioreactor unit. Otherwise, hepatitis virus can be constructed in the hepatocyte. Infectious cDNA clone of hepatitis virus can also be injected. In accordance with the invention, thus, "infection with hepatitis virus" includes not only infection with complete hepatitis virus particle but also infection with an infectious recombinant vector expressing a nucleic acid constructing hepatitis virus in hepatocyte. After the hepatitis virus-containing liquid culture medium is supplied, further, the supply of fresh one of the liquid culture medium or the circulation of the liquid culture medium is stopped, preferably for about 2 to 24 hours, more preferably for about 2 to 10 hours after the supply of the hepatitis virus-containing liquid culture medium; and while fresh one of the liquid culture medium is not supplied preferably for about 2 to 48 hours, more preferably for about 6 to 48 hours thereafter, the liquid culture medium discharged from the top of the main bioreactor unit 12 is preferably supplied again as a liquid culture medium into the main bioreactor unit 12. In such manner, the infection possibility of hepatitis virus can be increased. For about 15 minutes to 4 hours, preferably about 30 minutes to 2 hours immediately prior to the infection with hepatitis virus, additionally, the velocity of fresh liquid culture medium supply and the velocity of oxygen supply till then are preferably increased to about 1.5-fold to 4-fold, preferably about 1.5-fold to 2.5-fold for culture. In such manner, the possibility of infection with hepatitis virus can be increased, while the cell state can be retained well. After the start of hepatocyte culture, just when the oxygen consumption in ppm amounts to about half of the volume in ml of the main bioreactor unit ± 30 % (for example, 15 ppm ± 30 % in case that the volume of the main bioreactor unit is 30 ml), the culture temperature is gradually lowered to stabilize the oxygen consumption. After the oxygen consumption has become stable, the infection with the virus as described above is preferable so as to increase the possibility of virus infection. In that case, the culture temperature is preferably 28°C to 34°C, more preferably about 29°C to 32°C. The culture after the virus infection is preferably carried out at such low temperature, so as to sustain the virus infection and retain the cell state well.

After the treatment for the hepatitis virus infection, the culture of the hepatocyte is continued under the conditions described above, so that the hepatitis virus proliferates in the hepatocyte and is then contained in the liquid culture medium discharged from the liquid culture medium discharge pipe 20, in about 2 to 3 weeks post-infection. By subsequently recovering the hepatitis virus from the discharged liquid culture medium , the hepatitis virus can be isolated. The isolation of the hepatitis virus from the liquid culture medium can be done by general methods using filtration using ultrafiltration membrane, centrifugation, gel permeation chromatography and the like.

The method of the invention discloses that even a cell with low adhesivity, such as hepatocyte, can be developed and cultured in a three dimension in the same fashion as in biological organisms, at a mass scale for a long term. Thus, the invention provides a method for culturing a cell with low adhesivity in a three dimension at a mass scale for a long term.

Additionally, the method of the invention is applicable not only to HCV described above but also hepatitis viruses such as HBV and HEV and Dengue fever virus in hepatocyte.

The virus proliferated by the method of the invention can be utilized as an immunogen for vaccine development and anti-hepatitis virus antibody induction. The proliferation system of hepatitis virus in the hepatocyte during culture can be utilized not only for recovering hepatitis virus but also for developing therapeutic agents of hepatitis, such as protease inhibitor, antisense RNA or antisense DNA or the like.

### Examples

The invention will now be described more specifically with reference to examples. Herein, the invention is never limited to the following examples.

### Example 1

### (1) Radial flow type bioreactor

A radial flow type bioreactor of the structure shown in Fig. 1 was prepared. Main bioreactor unit 12 and liquid culture medium discharge pipe 20 were made of a porous metal-sintered material with a through-hole of a diameter of about 40 µm. The inner volume of the main bioreactor unit 12 was 30 ml. The porous carrier filled in the inside of the main bioreactor unit 12 was a porous glass bead (trade name of Siran; Schott Glasswerk Co. Ltd., Germany). The glass bead was of a diameter of 0.6 mm, where pores were formed in a honeycomb shape in the inside thereof. The glass bead was at a void ratio of 50 % and with a surface area of 90 m2/L-matrix and pore diameters of 20 to 120 µm. 18 g of such glass bead was filled in the main bioreactor unit 12. Thus, the cell adhesion area was 1500 cm²/g-matrix.

### (2) Culture system

The schematic outline of a culture system including the radial flow type bioreactor described above in (1) is illustratively shown in Fig. 2. In the system shown in Fig. 2, a fresh liquid culture medium is reserved in fresh liquid culture medium reservoir 24 and is transferred with pump 26 into liquid culture medium adjustment vessel 28. Agitator 30 is arranged on the liquid culture medium adjustment vessel 28, where the oxygen concentration, carbon dioxide concentration and pH of the liquid culture medium are adjusted. Additionally, a heating device is arranged on table 31 where the liquid culture medium adjustment vessel 28 is mounted, and the heating device can adjust the temperature of the liquid culture medium. In NaOH reservoir 32 is reserved IN NaOH, which is transferred, if necessary, with pump 34 in the liquid culture medium adjustment vessel 28, to adjust the pH of the liquid culture medium. Alternatively, oxygen bomb 36 and carbon dioxide bomb 38 are connected through flow controller 40 to the liquid culture medium adjustment vessel 28. From the oxygen bomb 36 and the carbon dioxide bomb 38 are supplied necessary volumes of oxygen and carbon dioxide, respectively, to the liquid culture medium adjustment vessel 28. The flow controller 40 is connected to microcomputer 42, and the microcomputer checks and controls the flows at a frequency of once per 20 minutes. The liquid culture medium with the oxygen concentration, carbon dioxide concentration and pH as adjusted in the liquid culture medium adjustment vessel 28 is supplied through pump 44 to the radial flow type bioreactor 10 from the bottom thereof. As described with reference to Fig. 1, the liquid culture medium supplied is discharged from the top of the liquid culture medium discharge pipe of the radial flow type bioreactor 10. The discharged liquid culture medium is reserved through pump 46 in liquid culture medium discharge reservoir 48. In the culture system shown in Fig. 2, further, a conduit for recycling the discharged liquid culture medium to the culture medium adjustment vessel 28 is arranged, so that the culture system structurally can again supply the whole or a part of the discharged liquid culture medium as a liquid culture medium, on a needed basis. Not shown in the figure, the microcomputer 42 is connected to each of the pumps, and is also connected to an oxygen concentration meter not shown in the figure to assay the liquid culture medium to be supplied to the main bioreactor unit and to an oxygen concentration meter not shown in the figure to assay the oxygen concentration of the discharged liquid culture medium. The microcomputer 42 is additionally connected to a pH meter and a thermometer, not shown in the figure, as arranged in the liquid culture medium adjustment vessel 28. The microcomputer 42 automatically controls the oxygen concentration, pH and temperature of the liquid culture medium and the supply of the liquid culture medium.

### (3) Inoculation and culture of hepatocyte

The FLC-4 line as an established hepatocyte line was subcultured in a flask to 2 × 10⁹ cells. After the liquid culture medium flowed in the main bioreactor unit, the FLC-4 cell cultured in the flask was added to the liquid culture medium and was then supplied into the main bioreactor unit. In such manner, hepatocyte was inoculated. Over 6 hours post-inoculation, the pumps 44 and 46 were stopped to prevent the efflux of the cell from the main bioreactor unit. Thereafter, fresh one of the liquid culture medium was supplied at a flow of 25 ml/day. Additionally, the circulation velocity of the liquid culture medium was 10 to 40 L/day. The liquid culture medium was at pH 7.0 and a temperature of 37°C, while the oxygen concentration therein was automatically controlled with a computer, so that the oxygen concentration in the discharged liquid culture medium could be 1 to 6 ppm.

The liquid culture medium was commercially available and was of the following composition (all are expressed in unit mg/L).

| | |
|---|---|
| NaCl | 6000 |
| MgCl₂ | 100 |
| NaH₂PO₄ | 125 |
| ZnSO₄-7H₂O | 0.01 |
| D-Glucose | 2000 |
| L-Aspartate hydrochloride salt | 200 |
| L-Alanine | 20 |
| L-Aspartic acid | 20 |
| Glycine | 30 |
| L-Isoleucine | 105 |
| L-Phenylalanine | 67 |
| L-Ornithine | 100 |
| L-Tryptophan | 25 |
| L-Valine | 94 |
| Uridine | 5 |
| Folic acid | 4 |
| Pyridoxal hydrochloride | 4 |
| Sodium pyruvate | 110 |
| HEPES | 1200 |
| Human transferrin | 5 |
| Phenol red | 5 |
| KCl | 400 |
| MgSO₄ | 98 |
| FeSO₄-7H₂O | 0.8 |
| CuSO₄-5H₂O | 0.001 |
| D-mannose | 500 |
| D-Galactose | 200 |
| Alanyl-L-glutamine | 500 |
| L-Glutamate | 20 |
| Glycyl-L-glutamine | 500 |
| L-Lysine | 146 |
| L-Serine | 80 |
| L-Threonine | 95 |
| L-Tyrosine | 64 |
| Succinic acid | 106 |
| Choline bitartarate | 20 |
| Inositol | 20 |
| Riboflavin | 0.4 |
| Glycerophosphoric acid | 1500 |
| NaHCO₃ | 1800 |
| Insulin | 5 |

The liquid culture medium, to which was added calf fetus albumin at 2 %, was used. The cell activity could be confirmed on the basis of the increase in the consumption of oxygen and glucose in the liquid culture medium. The oxygen consumption was observed to increase smoothly. Herein, furthermore, the oxygen consumption was calculated on the basis of the difference between the oxygen concentration in the liquid culture medium supplied to the main bioreactor unit 12 and the oxygen concentration in the liquid culture medium discharged from the top of the main bioreactor unit 12. Additionally, the glucose concentration in the discharged liquid culture medium was assayed, using a commercially available glucose concentration assay kit. Further, the glucose consumption was calculated on the basis of the difference between the assay concentration and the glucose concentration in the supplied liquid culture medium.

### (4) HCV infection

Just when the oxygen consumption reached 15 ppm on day 7 from the start of the culture in the reactor, the culture temperature was gradually lowered to 32°C. On day 9 from the start of the culture, when the oxygen consumption by the cell was stabilized, HCV infection was done. Prior to HCV infection, the supply volumes of the liquid culture medium and oxygen were increased to 2-fold, for one-hour culture. Thereafter, HCV infection was done. HCV infection was carried out by dissolving one ml of the serum of a patient with chronic type C hepatitis (an infection titer of 5.5 CID50/ml for chimpanzee) in 10 ml of the liquid culture medium and using the resulting liquid culture medium as the liquid culture medium to be supplied into the main bioreactor unit, through a pipe not shown in the figure, which is branched from a liquid culture medium circulation tube immediately following the top of the main bioreactor unit and is in communication with the inside of the main bioreactor unit. Over 6 hours since then, the pump was stopped. Starting circulation pump 44, then, the whole volume of the liquid culture medium discharged from the top of the bioreactor 10 was recycled into the liquid culture medium adjustment vessel 28. Without any fresh supply of such culture medium, HCV was cultured for 24 hours. Subsequently, the culture was continued in the same manner as described above (herein, the culture temperature was 29 to 32°C).

### (5) HCV detection in discharged liquid culture medium

After the treatment for HCV infection, the culture was again started under the general conditions described above. Then, HCV was detected by a general method RT-PCR.

Herein, the nucleotide sequence of the primer for reverse transcription was as follows:

AACACTACTCGGCTAGCAGT

Additionally, the nucleotide sequences of primers for PCR were as follows. First primers are CTGTGAGGAACTACTGTCTT and AACACTACTCGGCTAGCAGT Second primers are TTCACGCAGAAAGCGTCTAG and

GTTGATCCAAGAAAGGACCC.

Further, PCR was carried out for a total volume of 50 µl at 35 cycles of a denaturation step at 94 °C for 45 seconds, an annealing step at 55 °C for 45 seconds and an extension step at 70 °C for 60 seconds.

Consequently, HCV was detected on day 1 to day 2 after the infection treatment, but thereafter, no HCV was detected. On day 16 and thereafter, HCV was again detected. On day 19 post-infection, HCV reached maximum at 10⁴ to 10⁵ copies /ml.

HCV RNA was consistently detected throughout the entire 100-day period post-infection. The reason why HCV was detected on day 1 to day 2 post-infection treatment is believed to reside in that the HCV added and flowing out was detected. The reason why HCV was not detected on day 3 post-infection treatment and thereafter is believed to reside in that the efflux of HCV was terminated. The reason why HCV was again detected on day 16 and thereafter is believed to reside in that HCV after infection of hepatocyte was proliferated in the hepatocyte and the resulting proliferated HCV was released into the liquid culture medium.

### (6) Nucleotide sequence of HCV

The nucleotide sequence of the HVR (hyper variable region) of the virus detected on day 23 post-infection was examined (see Table 1 hereinbelow). The clone occupying the bioreactor mostly as much as 95 % was identical to the major clone A1 occupying originally 55 to 60 % of the serum of the patient.

The results are shown in Table 1, so that the results can be compared with a blood transfusion example and a chimpanzee example. "RFB (radial flow bioreactor)" in Table 1 represents the results of the experiment. Herein, the sequences are shown as sequences according to the one-character amino acid expression method.

The "clone number" in Table 1 represents the numbers of clones recovered from the sera of a donor, chimpanzee and a recipient and the number of clones recovered from RAD. The numbers of clones recovered from the sera of the blood donor and chimpanzee are based on the data of Aizaki et al.

"W" in Table 1 represents the period in week post-blood transfusion, while "D" represents the period in day post-infection. The numerical figures on the amino acid sequences in Table 1 represent the positions of the amino acids in HCV protein, while the hyphen represents an amino acid identical to the amino acid described on the utmost top.

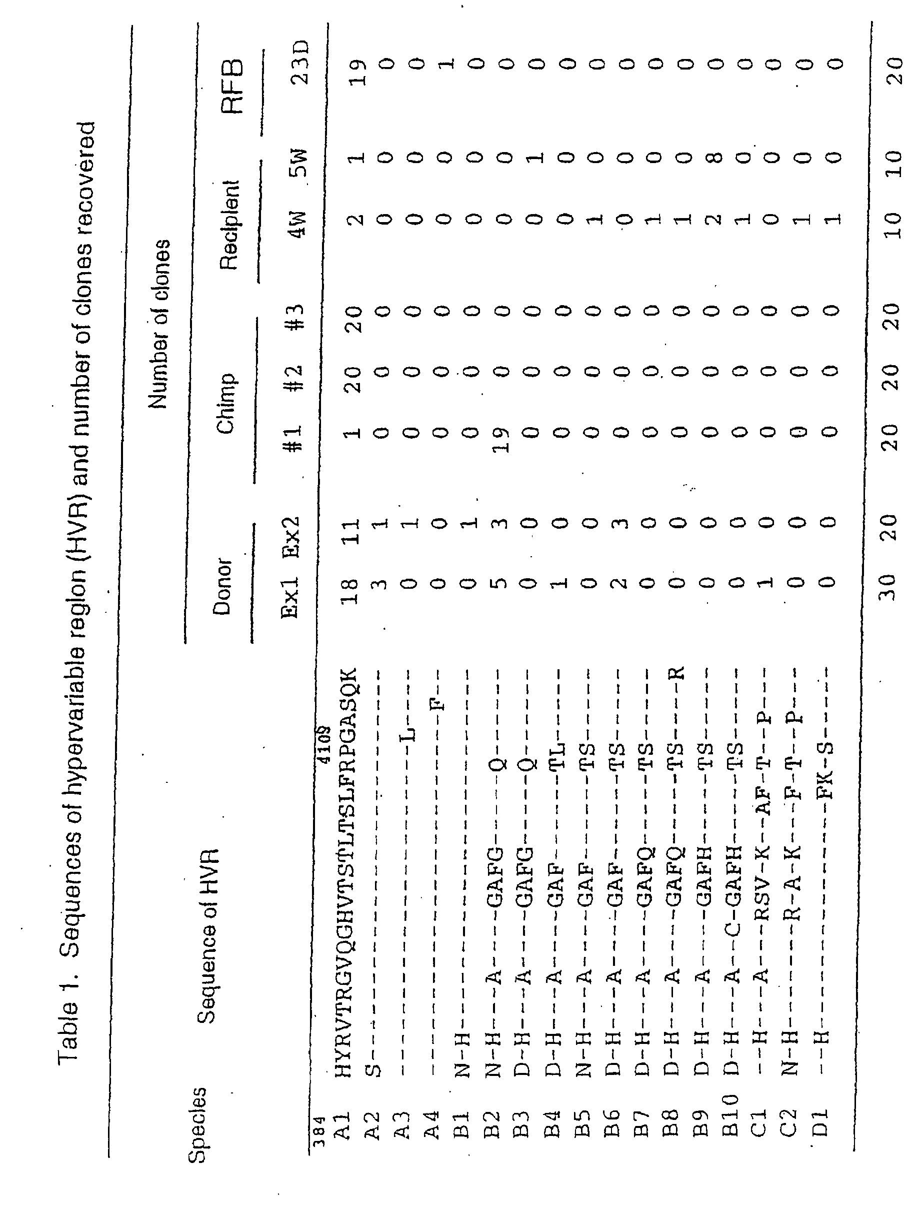

### Example 2

The same experiment as in Example 1 was carried out under modified conditions.

### (1) Culture system for serum-added liquid culture medium

The same bioreactor as in Example 1 was used. FLC-4 cell cultured in the flask to 1 × 10⁹ cells was inoculated in a culture medium vessel under control. Using a 2 % serum-added culture medium (liquid culture medium) at 50 ml/day, the FLC-4 cell was cultured. Then, the oxygen consumption of the FLC-4 cell in the bioreactor was gradually increased. On day 30, then, the oxygen consumption amounted to 25 ppm (see Fig. 3(A)) and on day 105, the oxygen consumption amounted to 35 ppm. So as to block that the dissolved oxygen concentration in the bioreactor backward might be below 1.0 ppm during the course, the culture temperature was lowered gradually from 37°C (see Fig. 3(A)). On day 105, the temperature was lowered to 30°C. The albumin in the liquid culture medium was above 75 µg/ml during the course of such low-temperature culture, so the activity of the cell was retained.

Fig. 3(A) depicts the changes of temperature (°C), oxygen concentration (ppm) and albumin concentration (µg/ml) during the culture. In Fig. 3 (A), open circle symbol (○) represents temperature (°C); solid circle symbol (●) represents oxygen concentration (ppm); and open square symbol (□) represents albumin concentration (µg/ml). The temperature (°C) and oxygen concentration (ppm) are depicted on the left scales, while the albumin concentration (µg/ml) is depicted on the right scale. The period in day is shown on the crosswise axis. The numerical figure is as shown in Fig. 3(C).

By the same method as in Example 1(5) described above, HCV in the discharged liquid culture medium was detected.

The results are shown in Fig. 3 (B). In Fig. 3(B), open circle symbol (○) represents RNA titer; open square symbol (□) represents that HCV core protein is negative; and solid square symbol (■) represents that HCV core protein is positive. The longitudinal axis in Fig. 3(B) depicts RNA titer (log₁₀ copy number/ml). The culture period in day is shown on the crosswise axis.

Fig. 3(C) depicts the changes of GPT (IU/1), GOT (IU/1) and LDH (IU/1). In Fig. 3 (C), solid circle symbol (●) represents GPT (IU/1); open circle symbol (○) represents GOT (IU/1); and open square symbol (□) represents LDH (IU/1). GPT (IU/1) and GOT (IU/1) are depicted on the left scales, while LDH (IU/1) is depicted on the right scale. The period in day is depicted on the crosswise axis.

### (2) Culture system for serum-free liquid culture medium

Using a serum-free culture medium (liquid culture medium) at 50 ml/day, the FLC4 cell was cultured in the bioreactor.

The results are shown in Figs. 4 and 5. Fig.5 depicts the case of transfection with the infectious clone RNA of type 1a in the bioreactor (see (4) below).

Fig. 4(A) and Fig. 5(A) depict the changes of temperature (°C), oxygen concentration (ppm) and albumin concentration (µg/ml) during culture. Herein, Fig. 4(A) never depicts albumin concentration (µg/ml). In Fig.4 (A) and Fig. 5(A), open circle symbol (○) represents temperature (°C) and solid circle symbol (●) represents oxygen concentration (ppm). In Fig. 5(A), the open square symbol (□) depicts albumin concentration (µg/ml). The temperature (°C) and oxygen concentration (ppm) are depicted on the left scales, and the albumin concentration (µg/ml) is depicted on the right scale. The period in day is shown on the crosswise axis. The numerical figures are as shown in Fig. 4(C) and Fig. 5(C).

By the same method as in Example 1(5) described above, HCV in each discharged liquid culture medium was detected.

The results are shown in Fig. 4(B) and Fig. 5(B). In Fig. 4(B) and 5(B), open circle symbol (○) represents RNA titer; open square symbol (□) represents that HCV core protein is negative; and solid square symbol (■) represents that HCV core protein is positive. The longitudinal axis in Fig. 4(B) and Fig. 5(B) depict RNA titer (log₁₀ copy number/ml). The culture period in day is shown on the crosswise axis.

Fig.4(C) and Fig. 5(C) depict the changes of GPT (IU/1), GOT (IU/1) and LDH (IU/1) in the liquid culture medium. In Fig. 4 (C) and Fig. 5 (C), solid circle symbol (●) represents GPT (IU/1); open circle symbol (○) represents GOT (IU/1); and open square symbol (□) represents LDH (IU/1). GPT (IU/1) and GOT (IU/1) are depicted on the left scales, while LDH (IU/1) is depicted on the right scale. The period in day is depicted on the crosswise axis.

For the culture system, the culture temperature was lowered to 35°C for culture, on day 5 after the start of the culture, so that the cell could be cultured without subculture for a long period as long as 100 days or more (see Fig. 5(A)). During the period, the oxygen consumption of the cell was stable at 15 to 20 ppm (see Fig. 5(A)).

As described above, the hepatoma-derived cell line could be cultured without subculture for a period as long as 100 days or more, by culturing in the serum-free culture medium or the 2 % serum added-culture medium at a low temperature of 30°C to 35°C, using the radial flow type bioreactor. During the period, hepatocyte activities as represented by oxygen consumption, glucose consumption and albumin level were retained.

### (3) Infection experiment with serum from chronic hepatitis patient

In the same manner as in Example 1(4), the cell was infected with the serum of a chronic hepatitis patient. HCV was detected in the discharged liquid culture medium, in the same manner as in Example 1(5). Consequently, the virus RNA turned once negative on day 3 post-infection but subsequently turned positive again. On day 10 post-infection, the virus reached maximum at 10³ to 10⁴ copies/ml (see Fig. 4(B)).

In the same manner as in the case of Example 1 described above, HCV RNA fell once negative after inoculation but was again increased to turn positive. Therefore, infection with the virus and the proliferation thereof were indicated. During the course at any of the infection experiments in Examples 1 and 2, additionally, no increase of markers of liver disorders, such as GOT, GPT and LDH, was observed (see Fig. 4(C) and Fig. 5(C)).

### (4) Transfection experiment with infectious clone

The transfection with the infectious clone RNA of type 1a was done in the bioreactor (see Fig. 5). Immediately after the transfection, the virus RNA was gradually decreased. On day 44, the virus number was less than 10² copies/ml. Subsequently on day 57, the virus number was increased again to 10⁴ copies/ml (see Fig. 5(B)) and was retained at about 10³ to 10⁴ copies/ml until day 100. Further, the transfection was conducted as follows: by the lipofection method, transfection with 10 µg of an infectious clone H77 (Yanagi et al., 1998, Proc Natl Acad Sci USA. Aug 5: 94(16): 8738-43) was done in the same manner as for the patient serum described above, while stopping the circulation tentatively. Herein, the transfection by the lipofection method was carried out, specifically, as follows.

10 µg of RNA in vitro synthesized from the H77 was mixed with 1 ml of OptiMEM and 150 ul of lipofectin, and the resulting mixture was left to stand at ambient temperature for 15 minutes. Subsequently, the inside of the reactor was sufficiently filled with OptiMEM, into which the RNA was injected.

Additionally, the core protein was gradually increased in the culture supernatant and reached maximum on day 44 (see Fig. 5(B)). The core protein was assayed by immunoassay. For more specific description, the virus particle in the liquid culture medium is precipitated with a precipitation reagent into a precipitate fraction, which is then collected and dispersed in a dispersion reagent. After treatment with an antibody reagent and a neutralization reagent, the HCV core protein binds to an anti-HCV core protein monoclonal antibody on the tube, to form a complex. After unreactive substances are rinsed off, a peroxidase-labeled anti-HCV core protein monoclonal antibody is added to the complex on the tube. Then, the monoclonal antibody binds to the complex. After unreactive substances are rinsed off, a solution of HPPA is added to generate a fluorescent substance via the peroxidase enzyme bound on the tube, followed by irradiation of an excitation beam of 323 nm to emit fluorescence, which is assayed at 410 nm. The concentration is calculated on the basis of a standard curve preliminarily prepared using standard solutions.

So as to confirm the presence of the core protein, further, the liquid culture medium on day 96 post-transfection was concentrated to 200-fold and was then fractionated by a method on a 10 - 60 W/W% sucrose gradient, to assay HCV RNA and HCV core protein in the individual fractions.

The results are shown in Fig. 6. In the graphs in the lower part of Fig. 6, open circle symbol (○) represents HCV RNA titer (log₁₀ copy number/ml); and solid circle symbol (●) represents the concentration (pg/ml) of HCV core protein. In the graphs in the upper part of Fig. 6, solid square symbol (■) represents the density (g/ml) of the core protein.

Consequently, the peak of the core protein was measured on the density gradient at two positions of about 1.07 and 1.18 g/ml. It is indicated that the core protein is shown on a curve with two peaks. This indicates the presence of the virus particle in the liquid culture medium.

Additionally, after the liquid culture medium on day 88 and day 44 post-transfection was treated with RNase, HCV RNA could be detected by nest-RT PCR. Thus, the presence of HCV RNA protected in the virus particle is revealed (see Fig. 7). The nucleotide sequences of the primers used for the nest-RT PCR are CTGTGAGGAACTACTGTCTT, AACACTACTCGGCTAGCAGT for the forward side; and TTCACGCAGAAAGCGTCTAG, GTGATCCAAGAAAGGACCC for the reverse side.

The results are shown in Fig. 7. Fig. 7 depicts the liquid cultures on the previous day of culture (day -1) and on days 8 and 44 during culture, a control serum and a control RNA are shown from the left, individually in three lanes, namely a lane without RNase treatment (RNase-) or no nest-RT PCR treatment (RT-), a lane without RNase treatment (RNase-) but with nest-RT PCR treatment (RT+) and a lane with RNase treatment (RNase+) and nest-RT PCR treatment (RT+). The numerical figure on the top of Fig. 7 represents the period of culture in day; the symbols +- depicted on the top represent the presence (+) and absence (-) of the nest-RT PCR treatment; and the symbols on the bottom represent the presence (+) and absence (-) of RNase treatment. The numerical figure in the longitudinal direction in Fig. 7 represents the number of bases (-mer).

Because minus chain RNA could be detected within the cell on day 110 post-transfection by the RT PCR method using a tag, the presence of a virus replication intermediate within the cell was suggested (see Fig. 8). The RT PCR was specifically carried out as follows. The nucleotide sequences of the primers for use in the RT PCR are TCTTGGTGGCGAATAAGCCATGGCGTTAGTAT, for reverse transcription reaction; and TCATGGTGGCGAATAA and CGCGGCAACAAGTAAA for the forward side and reverse side, respectively of PCR reaction.

The results are shown in Fig. 8. In Fig. 8, M depicts marker; N in the lane 1 depicts control with cell (-) where neither negative strand RNA nor positive strand RNA is present; (-) RNA in the lane 2 represents the case with addition of negative strand RNA and (+) RNA in the lane 3 represents the case with addition of positive strand RNA. Cell in the lane 4 represents the case of HCV-infected cell cultured in RFB. The numerical figure in the longitudinal direction in Fig. 8 represents molecular weight. Consequently, the presence of the negative strand RNA was observed in the cultured cell after infection.

The nucleotide sequence of HVR during the culture course was compared with the original infectious clone. On days 25, 71 and 106, mutations were observed about one base, 2 bases and 2 bases, respectively. Throughout the culture course, no tendency of convergence into a specific nucleotide sequence or no accumulation of mutation was observed. The results are shown in Table 2. The results are shown in the column "RFB (radial flow bioreactor)" in Table 2. Further, the sequences are expressed as sequences according to the one-character amino acid expression method.

The "clone number" in Table 2 represents the number of clones recovered from the liquid culture medium.

The "D" in Table 2 represents the period in day after infection.

The numerical figure above the amino acid sequence in Table 2 represents the position of the amino acid in HCV protein, and the hyphen represents an amino acid identical to the amino acid described on the utmost top.

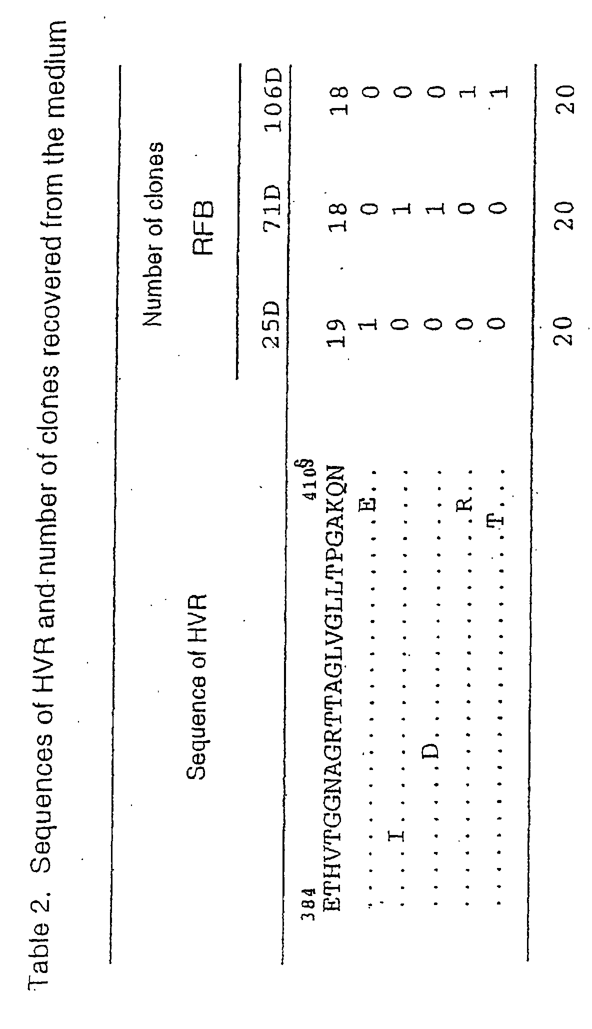

As described above, the transfection with the infectious clone of type 1a was carried out in the bioreactor. Successfully, HCV replication was confirmed in the following five ways: 1) the re-increase of the virus RNA; 2) the increase of the core protein; 3) the presence of the virus RNA in the particle; 4) the presence of the intracellular virus replication intermediate; and 5) the mutation of the nucleotide sequence. As to the liver disorders involved in the virus proliferation, no apparent increase of GOT, GPT or LDH was observed during the course (see Fig. 5(C)).

### Industrial Applicability

The invention provides for the first time the culture and proliferation method of hepatitis virus outside biological organisms, and hepatitis virus has been believed to involve difficulty in the culture thereof outside biological organisms. The invention provides materials not only for research works on hepatitis virus but also for research works and development of the therapeutics and prevention of hepatitis virus infectious diseases and the mechanism thereof. More specifically, the invention provides an approach for yielding the virus essential for research works and development of a therapeutic agent of viral hepatitis by simple procedures.

Further, the method of the invention provides a method for proliferating the virus, so as to elucidate the proliferation mechanism and mutation mechanism of the virus.

As described above, the invention provides a method for stably supplying a necessary quantity of the virus, not only for research works about the profile of the virus but also for the research works and development of a method for therapeutically treating, preventing and treating the viral infectious diseases.

## Claims

1. A method for proliferating a hepatitis virus, **characterized by**
making a liquid culture medium flow around the periphery of a carrier being placed in a culture vessel and capable of immobilizing a cell with low adhesivity thereon,
immobilizing and proliferating the cell with low adhesivity on the carrier, and
allowing the cell under culture to be infected with a hepatitis virus to proliferate the hepatitis virus.

2. A method according to claim 1, where the carrier is a particulate porous carrier.

3. A method according to claim 1 or 2, where the cell with low adhesivity is hepatocyte.

4. A method according to any one of claims 1 to 3, where the cell with low adhesivity is an established cell.

5. A method according to claim 5, where the hepatitis virus is HCV.

6. A method according to any one of claims 1 to 5, where the flow of the liquid culture medium around the periphery of the carrier is a flow from the outer periphery of the culture vessel toward the center thereof.

7. A method for proliferating a hepatitis virus, **characterized by**
allowing hepatocyte maintained in a radial flow type hepatocyte bioreactor to permit a liquid culture medium to flow from the periphery of the main bioreactor unit placing therein a particulate porous carrier immobilizing thereon the hepatocyte toward the center thereof, to be infected with a hepatitis virus, and
continuously allowing the liquid culture medium to flow from the periphery of the main bioreactor unit toward the center thereof to culture the hepatocyte to thereby proliferate the infectious hepatitis virus in the hepatocyte.

8. A method according to claim 7, where the hepatocyte is of an established cell line.

9. A method according to claim 8, where the established cell line is the FLC-4 line (FERM BP-5165).

10. A method according to any one of claims 7 to 9, where the infection with the hepatitis virus is carried out by adding the hepatitis virus to the liquid culture medium, the method being **characterized by**
a step of adding the hepatitis virus to the liquid culture medium and subsequently circulating the culture medium used under no supply of any fresh one of the culture medium, and
a step of subsequently stopping the flow of the liquid culture medium and circulating the culture medium used under no supply of fresh one of the culture medium.

11. A method according to any one of claims 7 to 10, **characterized in that** the supply velocity of fresh one of the culture medium and the supply velocity of oxygen are increased more than those velocities till then, prior to the addition of the hepatitis virus to the liquid culture medium.

12. A method according to any one of claims 7 to 11, where the hepatitis virus is hepatitis type C virus.

13. A proliferation apparatus of a hepatitis virus, **characterized in that** the apparatus is a radial flow type hepatocyte bioreactor having
a main bioreactor unit capable of allowing a liquid culture medium to flow from the periphery to the center thereof,
a liquid culture medium supply conduit supplying the liquid culture medium to the periphery of the main bioreactor unit,
a particulate porous carrier placed in the inside of the main bioreactor unit to immobilize hepatocyte thereon, and
a liquid culture medium discharge conduit positioned in the inside of the main bioreactor unit for discharging the liquid culture medium from the main bioreactor unit.

14. A proliferation apparatus according to claim 13, which is a proliferation apparatus of hepatitis type C virus.

15. A method for proliferating a cell with low adhesivity, **characterized by**
making a liquid culture medium flow around the periphery of a carrier capable of immobilizing thereon the cell with low adhesivity in a culture vessel placing therein the carrier, and
immobilizing and proliferating the cell with low adhesivity on the carrier.

16. A method according to claim 15, where the proliferation is three-dimensional proliferation.

17. A method according to claim 15 or 16, where the cell with low adhesivity is hepatocyte.

18. A method according to anyone of claims 15 to 17, where the cell with low adhesivity is an established cell.

19. A method according to any one of claims 15 to 17, where the hepatitis virus is HCV.

20. A method according to any one of claims 15 to 19, where the flow of the liquid culture medium around the periphery of the carrier is the flow from the outer periphery of the culture vessel toward the center thereof.
